# EUROPEAN PATENT SPECIFICATION

(11) **EP 2 586 528 B1**
(45) Date of publication and mention of the grant of the patent: **25.09.2024**
(21) Application number: 11797608.4
(22) Date of filing: 21.06.2011
(51) Int. Cl.: B01J 23/72, B01J 23/889, B01J 23/83, B01J 23/80, B01J 37/02, C07C 31/20, C07C 29/149

(54) **CATALYST HAVING MONOLITHIC STRUCTURE FOR MANUFACTURING ETHYLENE GLYCOL BY OXALATE HYDROGENATION, PREPARATION METHOD AND APPLICATION THEREOF**
KATALYSATOR MIT MONOLITHISCHER STRUKTUR ZUR HERSTELLUNG VON ETHYLENGLYKOL DURCH OXALATHYDROGENIERUNG, HERSTELLUNGSVERFAHREN DAFÜR UND ANWENDUNG DAVON
CATALYSEUR PRÉSENTANT UNE STRUCTURE MONOLITHIQUE POUR LA PRODUCTION D'ÉTHYLÈNEGLYCOL PAR HYDROGÉNATION D'OXALATE, SON PROCÉDÉ DE PRÉPARATION ET SON APPLICATION

(30) Priority: 24.06.2010 CN 201010207694
(43) Date of publication of application: 01.05.2013
(73) Proprietor: Tianjin University, Tianjin 300072 (CN)
(72) Inventor: MA, Xinbin, Tianjin 300072 (CN); ZHAO, Yujun, Tianjin 300072 (CN); WANG, Shengping, Tianjin 300072 (CN); LV, Jing, Tianjin 300072 (CN); WANG, Baowei, Tianjin 300072 (CN); LI, Zhenhua, Tianjin 300072 (CN); XU, Yan, Tianjin 300072 (CN)
(74) Representative: JD&P Patent Attorneys Joanna Dargiewicz & Partners
(86) International application number: PCT/CN2011/076038
(87) International publication number: WO 2011/160577

(56) References cited:
- CN-A- 1 830 566
- CN-A- 101 138 725
- CN-A- 101 318 138
- CN-A- 101 342 489
- CN-A- 101 445 426
- CN-A- 101 590 407
- CN-A- 101 879 448
- CN-A- 101 879 448
- US-A- 4 649 226

## Description

The invention relates to a preparation method of a monolithic structure catalyst for producing ethylene glycol by hydrogenation of dimethyl oxalate, DMO, and the application of the catalyst for producing ethylene glycol by hydrogenation of dimethyl oxalate in a fixed bed reactor.

Ethylene glycol (EG) is an important basic organic material. It was first made by Wurtz in 1859 through hydrogenation of ethylene acetate catalyzed by potassium hydroxide. During the World War I, ethylene nitrate was used as substitute of glycerin to produce explosive because it could lower the freezing point of glycerin. Soon afterward, several feasible processes were developed. Hydrogenation of dichloromethane was used in Germany; chlorohydrin was used as raw stuff in America for the rapid growth of application of antifreeze with the development of automobile industry in the 1920s; ethylene and ethylene oxide was introduced as material which accelerated the process since polyester was developed and this process is widely used around the world mainly in Shell, UCC and Halcon-SD at present. The production capacity of these three companies takes up 90% of the world, followed by Japan catalytic Chemical Company, the Dow Chemical company, Mitsui East Asia Company and ICI Company.

The process of ethylene oxide hydration has several defects, such as longer process, high mole ratio of water and ethylene oxide, large energy consumption and low selectivity of EG. With the exhausting of oil around the world, experts are paying close attention to the processes without oil especially to the ones that using cheap resource as raw stuff. The process using syngas as material is mainly divided into two categories: direct synthesis and indirect synthesis. The promising method mainly contains formaldehyde dimerization, formaldehyde electrochemical hydrogenation dimer method, formaldehyde hydrogen formylation, glycolic acid method, formaldehyde condensation method, oxalate method and etc. The process of oxalate hydrogenation has several advantages such as mild reaction condition and high selectivity which is the most promising process to realize industrialization.

The process includes two key technologies: oxalate synthesis and hydrogenation of oxalate. For the oxalate production technology, the oxalate is generated though a circulate catalytic process composed of coupling and regeneration with the attending of nitrite and CO, produced from coal gasification and the following separation process of pressure swing adsorption (PSA). DMO production process is a cycle system which has a mild reaction condition, good stability, high selectivity and low pollution. For the ethylene glycol production process: the produced oxalate is catalysis hydrogenation to ethylene glycol with hydrogen produced via PSA process. This process is a complex reaction system, mainly including the following reactions:

ROOCCOOR + 2H₂ → ROOCCH₂OH + ROH

ROOCCH₂OH + 2H₂→ HOCH₂CH₂OH + ROH

HOCH₂CH₂OH + H₂→ C₂H₅OH + H₂O

The process that on studying mainly use noble mental such as ruthenium as catalyst in liquid phase and copper as catalyst in gaseous phase. Because of the high pressure and the difficulty of the catalyst separation from liquid phase, people are concentration on hydrogenation in gaseous phase on copper catalyst. ARCO Company of America [US 4112245] uses CuCr as catalyst which runs for 460 h and the conversion of diethyl oxalate reached 100%, selectivity of ethylene glycol excesses 95%. But because of Cr has great harm on human body and environmental impact, research of Cr-free catalyst gradually become the trend of catalyst for hydrogenation of oxalic ester. Ube prepared CuMOₖBaₚOₓ catalyst using mixed ball milling process in 1985 and was recorded in the United States Patent [US 4551565]. Conversion of diethyl oxalate was 100% and yield of ethylene glycol reached as high as 97.7% on this catalyst. Ube reported a Cu/SiO₂ catalyst in patent [US 4585890] in 1986. With silica sol and copper ammonia solution as precursors and heated in the mixing state after mixing evenly to remove most of the water, resulted in the precipitation of copper and silicon oxide. After washing, drying and calcination, we prepared the catalyst. Conversion of diethyl oxalate was 100% and selectivity of ethylene glycol was 97.2% on the catalyst under reaction conditions of pressure 3 MPa, temperature 215°C, hydrogen ester ratio 30. American UCC [US 4677234, US 4628128, US 4649226] has done a lot of research n capper-based chromium-free catalysts. They inspected different supporters (Al₂O₃, SiO₂, La₂O₅ etc), additives (Ag, Mo, Ba etc.) and preparation methods on the effect of catalytic reactivity and selectivity. Yield of ethylene glycol was up to 95% and time on stream was 466 h on the catalyst. UCC [US 4628128] regarded the annular catalyst with a central hole as the ideal catalyst for oxalic ester hydrogenation to ethylene glycol. The special structure had better diffusion characteristics which can reduce local overheat. Fudan University reported mesoporous zeolite supported copper catalysts modified with additives such as magnesium, manganese, chromium, or aluminum in patent [CN 101455976]. Conversion of oxalic ester reached 100% and selectivity of ethylene glycol achieved 96% on the catalyst under reaction conditions of temperature 210°C, pressure 3 MPa, hydrogen ester ratio 180, space velocity 0.5 h⁻¹. Dan Jin coal reported alumina supported copper catalyst modified with additives such as zinc, manganese, magnesium and chromium in patent [101524646]. With reaction pressure 0.3-1 MPa, temperature 145-200°C, mass space velocity of oxalate ester under 0.1-0.6 h⁻¹, conversion of oxalic ester was higher than 99% and selectivity of ethylene glycol achieve above 90%. CN101879448 A discloses a monolithic structure catalyst and a method for producing ethylene glycol using said catalyst.

Researchers have achieved particular progress on catalyst used for hydrogenation of oxalic ester to ethylene glycol, but some problems still exist in further engineering scale up. Larger ratio of height to diameter of the catalyst bed will benefit the uniform distribution and easy control of the bed temperature, since the hydrogenation of oxalic ester to ethylene glycol is a temperature sensitive reaction. However, the increase in the height to diameter ratio will result in the increase of bed resistance. Additionally, the increase in particle size of the catalyst will easily cause local-pot overheat and enhanced side reactions. These factors severely restrict the industrialization process of hydrogenation of oxalic ester to ethylene glycol technology.

An object of the present invention is to provide monolithic structure catalyst for producing ethylene glycol by hydrogenation of dimethyl oxalate. Active components of copper dispersed uniformly in the catalyst coating and the coating with thin layer form is evenly attached to the pore surface of cordierite honeycomb with monolithic structure. It effectively reduces the resistance of internal diffusion and improves the activity and selectivity in the hydrogenation of oxalic ester to ethylene glycol.

An additional object of the invention is to provide a method for preparing a monolithic structure catalyst for producing ethylene glycol by hydrogenation of dimethyl oxalate. The method comprises following steps: first copper based catalyst powder is prepared by precipitation method, from which a catalyst slurry is further acquired, and then coated the catalyst slurry directly on the surface of the cordierite honeycomb support to form the copper supported monolithic structure catalyst. The method ensures high dispersion of active component copper in the catalyst coating and enhances catalytic activity and thermal stability.

Another object of the invention is to provide a method for producing ethylene glycol by hydrogenation of dimethyl oxalate using a monolithic structure catalyst. The monolithic catalyst is used for hydrogenation of oxalic ester to ethylene glycol instead of particle catalyst, as it can reduce the cost by greatly reducing the pressure drop of the catalyst bed and decreasing the depletion of the catalyst, resulting from the abrasion during packing and reaction process. The provided monolithic catalyst has high catalytic activity, low resistance, convenient and quick replacement, which will benefit the realization of large-scale engineering amplification.

The invention provides a monolithic structure catalyst for producing ethylene glycol by hydrogenation of dimethyl oxalate, the catalyst denoted as Cu/SiO₂/cordierite, comprising a carrier, an additive, and an active component, the carrier being cordierite honeycomb of 400 cells per square inch (cpsi) and having particle size φ 15 × 25mm, the additive being silicate , the active component being copper, and the copper and silicate being coated on the cordierite to form a coating layer, wherein

The silicate accounts for 5-90 wt. % of the cordierite , the copper accounts for 1-40 wt. % of the cordierite , and the copper accounts for 5-50 wt. % of the coating layer.

The catalyst is prepared according to the following steps:
a) dissolving 15.5g of a soluble copper precursor Cu (NO₃)₂·5H₂O with 150 mL water to yield 0.37 M solution A ;
b) employing 25 wt.% ammonia aqueous solution as a precipitant and mixing with the solution A, the copper ammonia complex;
c) adding silicate precursor, 45 mL of 30 wt. % silica sol, into the solution A, stirring for 4 hours, heating to 95°C for precipitating the copper and additive, stopping heating when a pH of the solution is less than 7, filtering, washing with deionized water for 3 times, drying at 120°C for 12 hours, and calcinating a resulting precipitate at 450°C for 4 hours to obtain a catalyst powder B;
d) squeezing, granulating, and sieving part of the catalyst powder B to form a granular catalyst C having 40-60 meshes, mechanically mixing another part of catalyst powder B in quantity of 8.0g, 8.0g of the squeezed granular catalyst C, 0.5g of pseudoboehmite , and 50mL of water to form a catalyst slurry D , mixing time being 2 hours, rotating speed being 200 rpm to get the catalyst slurry D;
e) impregnating cordierite honeycomb of 400 cpsi, having particle size φ 15 × 25mm, in the catalyst slurry D for 5 min, blowing off the extra slurry from the carrier and then coating the catalyst slurry D onto the cordierite honeycomb using a dip coating method, drying at 120°C for 12 hours, and calcinating at 450°C for 6 hours to form a monolithic catalyst E.

The prepared monolithic catalyst Cu/SiO₂/cordierite is placed in a fix-bed reactor and reduced by 20% H₂/N₂ under the following conditions: hydrogen flow rate 50 mL/min, temperature 250°C for 20 h; after reduction, the system is filled with pure hydrogen and controlled under the following conditions: temperature 250°C and pressure 2.0 MPa, 20 wt. % DMO in methanol is pumped into the system; the LHSV of DMO is controlled at 1.5⁻¹ and the molar ratio of H₂/DMO is 150.

This invention further provides a method for preparing a monolithic structure catalyst denoted as Cu/SiO₂/cordierite for producing ethylene glycol by hydrogenation of dimethyl oxalate, the method comprising the steps of:
a) dissolving 15.5 g of a soluble copper precursor Cu (NO₃)₂·5H₂O with 150 mL water to yield a 0.37 M solution A;
b) employing 25 wt.% ammonia aqueous solution as a precipitant and mixing with the solution A ;
c) adding silicate precursor, 45 mL of 30 wt. % silica sol, into the solution A, the copper ammonia complex , stirring for 4 hours, heating to 95 °C for precipitating the copper and silicate , stopping heating when a pH of the solution is less than 7, filtering, washing with deionized water for 3 times, drying at 120°C for 12 hours, and calcinating a resulting precipitate at 450°C for 4 hours to obtain a catalyst powder B;
d) squeezing, granulating, and sieving part of the catalyst powder B to form a granular catalyst C having 40-60 meshes, mechanically mixing another part of catalyst powder B in quantity of 8.0g, 8.0g of the squeezed granular catalyst C, 0.5g of pseudoboehmite , and 50mL of water to form a catalyst slurry D, mixing time being 2 hours, rotating speed is 200 rpm;
e) impregnating cordierite honeycomb of 400 cpsi, having particle size φ 15 × 25mm, in the catalyst slurry D for 5 min, blowing off the extra slurry from the carrier and then coating the catalyst slurry D onto the cordierite honeycomb using a dip coating method, drying at 120 °C for 12 hours, and calcinating at 450°C for 6 hours to form a monolithic catalyst E denoted as Cu/SiO₂/cordierite.

This invention provides a method for producing ethylene glycol by hydrogenation of an oxalate using a monolithic structure catalyst, which comprises:
putting the monolithic structured catalyst of type Cu/SiO₂/cordierite into a fixed bed reactor, performing a reduction reaction in the presence of 20% H₂/N₂ at 250°C for 20 hours with hydrogen flow rate 50mL/min, after reduction introducing pure hydrogen into the reactor, maintaining a reaction temperature at 250 °C and a reaction pressure at 2.0 MPa, vaporizing methanol solution comprising 20 wt. % DMO in an evaporator, preheating, and introducing 20 wt.% DMO into the reactor, the liquid hourly space velocity (LHSV) of DMO being 1.5 g. mL⁻¹.h⁻¹, and a molar ratio of H₂ /ester 150.

Compared with the well known conventional technology, advantages of the invention are summarized below:
The copper based monolithic catalyst in this invention is used in the hydrogenation of dimethyl oxalate to ethylene glycol for the first time, which provides a new approach for the preparation of the catalyst used for the hydrogenation of dimethyl oxalate to ethylene glycol.

Compared with granular catalysts, the monolithic catalyst in this invention shortens the internal diffusion path, improves the gas-solid mass transfer efficiency, and increases the effective contact area between reactant and catalyst. As a result, the catalyst increases the reactivity and selectivity of EG.

In the monolithic catalyst provided by this invention, the active component copper is dispersed uniformly in the coating layer supported on the honeycomb carrier. Thus, the catalyst has higher thermal stability.

The monolithic catalyst in this invention has lower bed resistant than granular catalyst, so it can work at the conditions of larger height-diameter ratio and high hydrogen-ester ratio. As a result, the catalytic performance and temperature distribution are improved, and the hot-spot temperature is decreased effectively.

Compared with granular catalyst, the monolithic catalyst in this invention has high conversion of DMO and selectivity of EG in the hydrogenation of DMO to ethylene glycol, and it doubles the LHSV which presents higher production capacity.

The monolithic catalyst in this invention runs for 500 hours stably in the hydrogenation of DMO to ethylene glycol, average conversion of DMO reaches 100% and selectivity of EG is higher than 96%, which presents high hydrogenation activity and stability.

Compared with granular catalysts, the monolithic catalyst in this invention is more convenient to be packed and replaced.

The monolithic catalyst in this invention contains no Cr and other toxic element, which is environmentally friendly.

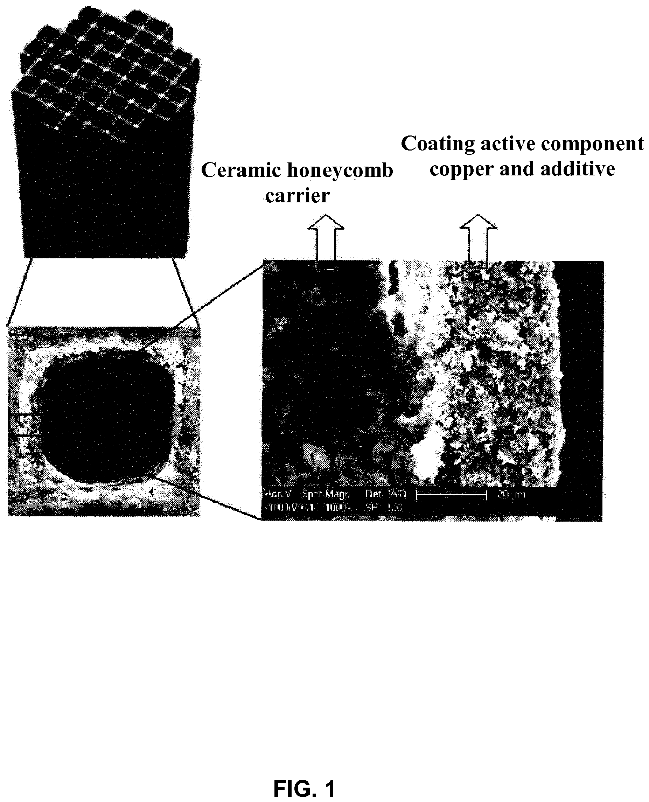

FIG. 1 shows an SEM image of a radial section of a monolithic catalyst; and

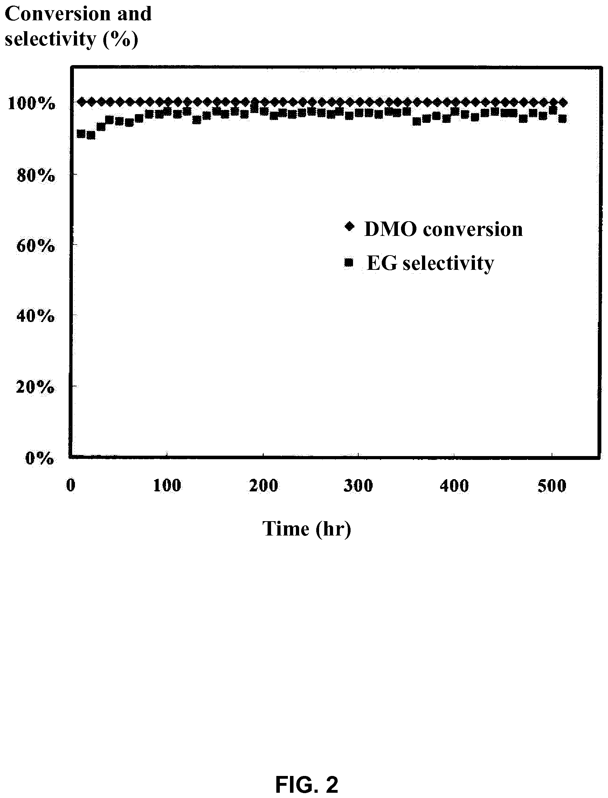

FIG. 2 shows stable data of a monolithic structure catalyst for producing ethylene glycol by hydrogenation of an oxalate.

For further illustrating the invention, experiments detailing a monolithic catalyst, a method for preparing and applying the same are described below. It should be noted that the following examples are intended to describe and not to limit the invention.

### Example 1

### Preparation of monolithic catalyst

15.5 g of Cu(NO₃)₂·5H₂O was dissolved in 150 mL water. 52 mL of 25 wt. % ammonia aqueous solution was added. Then 45 mL of 30 wt. % silica sol was added to the copper ammonia complex solution and aged by stirring for another 4 hours. The temperature was raised to 95°C to allow for the precipitation of copper and silicate. The filtrate was washed with deionized water for 3 times, dried at 120°C for 12 hours and calcined at 450°C for 4 hours to form catalyst powder with Cu content of 20 wt. %.

Part of the catalyst powder was squeezed and sieved to 40-60 meshes. 8.0 g of squeezed catalyst, 8.0 g of catalyst powder, 0.5 g of pseudoboehmite and 50 mL of water was added in the ball mill can to ball mill at 200 rpm for 2 hours to get the catalyst slurry.

Cordierite carrier (Φ15 × 25 mm) of 400 cpsi was impregnated in the slurry for 5 min, then extra slurry on the carrier was blew off and dried at 120°C for 12 hours, the coated cordierite carrier was then weighed. The above operation was repeated until the content of coat reached 20 wt. %. The as prepared catalyst was calcined at 450°C for 6 hours to get the monolithic catalyst denoted as Cu/SiO₂/cordierite.

### Catalytic activity test

The monolithic catalyst prepared in Example 1 is placed in the fix-bed reactor and reduced by 20% H₂/N₂ under the following conditions: hydrogen flow rate 50 mL/min, temperature 250°C for 20 h. After reduction, the system is filled with pure hydrogen and controlled under the following conditions: temperature 250°C and pressure 2.0 MPa. 20 wt. % DMO in methanol is pumped into the system. The LHSV of DMO is controlled at 1.5⁻¹ and the ratio of H₂/DMO is 50. C_{DMO} and S_{EG} are calculated from timing analysis results of production components with GC. The result is listed in Table 1.

**Table 1 Performance of catalysts at different ratios of H₂/DMO**

| | Ratio of H₂/DMO | C_{DMO} /% | S_{EG} /% |
|---|---|---|---|
| Example **1** | 50 | 100 | 93 |
| Example **2** | 150 | 100 | 96 |
| Example **3** | 200 | 100 | 91 |

### Example 2

The implementary conditions are the same as Example 1 except that that the ratio of H₂/DMO is 150. The result is listed in Table 1.

### Example 3

The implementary conditions are the same as Example 1 except that that the ratio of H₂/DMO is 200. The result is listed in Table 1.

### Comparative Example 1

15.5 g of Cu(NO₃)₂ ·5H₂O was dissolved in 150 mL water. 52 mL of 25 wt. % ammonia aqueous solution was added. Then 45 mL of 30 wt. % silica sol was added to the copper ammonia complex solution and aged by stirring for another 4 hours. The temperature was raised to 95°C to allow for the precipitation of copper and silica. The filtrate was washed with deionized water for 3 times, dried at 120°C for 12 hours and calcined at 450°C for 4 hours to form catalyst powder with Cu content of 20 wt. %.

The above Cu/SiO₂ catalyst powder is extruded to Φ5 × 4 mm particles. 2.00 mL as prepared catalyst is reduced by 20% H₂/N₂ with hydrogen flow rate of 50 mL/min in the fixed bed reactor at 350°C for 4 hours. After reduction, the system is filled with pure hydrogen and controlled under the following conditions: temperature 200°C and pressure 2.5 MPa. 20 wt. % DMO in methanol is pumped into the system. The LHSV of DMO is controlled at 0.4h⁻¹ and the ratio of H₂/DMO is 90. C_{DMO} and S_{EG} are calculated from timing analysis results of production components with GC. The result is that C_{DMO} is 98% and S_{EG} is 90%.

The monolithic catalyst provided by the invention is applied in the synthesis process of EG via hydrogenation of DMO. Compared with supported granular catalyst (Comparative Example 1), the monolithic catalyst shows a better performance in C_{DMO} and S_{EG}. Furthermore, the technology using monolithic catalyst can get a larger production of EG due to that LHSV of DMO and STY of EG are twice the conventional supported catalyst.

## Claims

1. A method for preparing a monolithic structure catalyst for producing ethylene glycol by hydrogenation of dimethyl oxalate, comprising the steps of:
a) dissolving 15.5g of the soluble copper precursor Cu (NO₃)₂·5H₂O with 150 mL water to yield 0.37 M solution A;
b) employing 25 wt.% ammonia aqueous solution as the precipitant and mixing with the solution A which is the copper ammonia complex;
c) adding the silicate precursor, 45 mL of 30 wt. % silica sol, into the solution A, the copper ammonia complex, stirring for 4 hours, heating to 95°C for precipitating the copper and silicate, stopping heating when a pH of the solution is less than 7, filtering, washing with deionized water for 3 times, drying at 120°C for 12 hours, and calcinating a resulting precipitate at 450°C for 4 hours to obtain the catalyst powder B;
d) squeezing, granulating and sieving part of the catalyst powder B to form the granular catalyst C having 40-60 meshes, mechanically mixing another part of catalyst powder B in quantity of 8.0g, 8.0g of the squeezed granular catalyst C, 0.5g of pseudoboehmite, and 50mL of water to form the catalyst slurry D, mixing time being 2 hours, rotating speed is 200 rpm;
e) impregnating cordierite honeycomb of 400 cpsi, having particle size φ 15 × 25mm, in the catalyst slurry D for 5 min, blowing off the extra slurry from the carrier and then coating the catalyst slurry D onto the cordierite honeycomb using a dip coating method, drying at 120°C for 12 hours, and calcinating at 450°C for 6 hours to form the monolithic catalyst E denoted as Cu/SiO₂/cordierite.

2. A method for producing ethylene glycol by hydrogenation of dimethyl oxalate, DMO, using the monolithic structure catalyst prepared by the method of claim 1, the method comprising the steps of:
putting monolithic structure catalyst of type Cu/SiO₂/cordierite into a fixed bed reactor, performing a reduction reaction in the presence of 20% H₂/N₂ at 250° C for 20 hours with hydrogen flow rate 50mL/min, after reduction introducing pure hydrogen into the reactor, maintaining a reaction temperature at 250°C, and a reaction pressure at 2.0 MPa, vaporizing methanol solution comprising 20 wt. % DMO in an evaporator, preheating, and introducing 20 wt.% DMO into the reactor, the liquid hourly space velocity (LHSV) of DMO being 1.5 g.mL⁻¹.h⁻¹, and a molar ratio of H₂/ester being 150.

## Patentansprüche

1. Verfahren zur Herstellung eines Katalysators mit monolithischer Struktur zur Herstellung von Ethylenglykol durch Hydrierung von Dimethyloxalat, umfassend die Schritte:
a ) Auflösen von 15,5 g des löslichen Kupfervorläufers Cu(NO₃)₂ 5H₂O mit 150 ml Wasser, um eine 0,37 M Lösung A zu erhalten;
b) Verwenden von 25 gew.-%iger wässriger Ammoniaklösung als Fällungsmittel und Vermischen mit der Lösung A, die der Kupfer-Ammoniak-Komplex ist;
c ) Hinzufügen des Silikatvorläufers, 45 ml 30 Gew.-% Silicasol, in die Lösung A, den Kupfer-Ammoniak-Komplex, Rühren für 4 Stunden, Erhitzen auf 95°C zur Ausfällung des Kupfers und des Silikats, Beenden des Erhitzens, wenn der pH-Wert der Lösung weniger als 7 beträgt, Filtrieren, dreimaliges Waschen mit entionisiertem Wasser, Trocknen bei 120°C für 12 Stunden und Kalzinieren eines resultierenden Niederschlags bei 450°C für 4 Stunden, um das Katalysatorpulver B zu erhalten;
d) Quetschen, Granulieren und Sieben eines Teils des Katalysatorpulvers B, um den körnigen Katalysator C mit 40-60 Maschen zu bilden, mechanisches Mischen eines anderen Teils des Katalysatorpulvers B in einer Menge von 8,0 g, 8,0 g des gequetschten körnigen Katalysators C, 0,5 g Pseudoböhmit und 50 ml Wasser, um die Katalysatoraufschlämmung D zu bilden, wobei die Mischzeit 2 Stunden beträgt und die Drehgeschwindigkeit 200 U/min beträgt, und
e) Imprägnieren von Cordierit-Honigwaben von 400 cpsi mit einer Teilchengröße von Φ 15 × 25 mm in der Katalysatoraufschlämmung D für 5 min, Abblasen der überschüssigen Aufschlämmung vom Träger und anschließendes Auftragen der Katalysatoraufschlämmung D auf die Cordierit-Honigwaben unter Verwendung eines Tauchbeschichtungsverfahrens, Trocknen bei 120°C für 12 Stunden und Kalzinieren bei 450°C für 6 Stunden, um den monolithischen Katalysator E, bezeichnet als Cu/SiC₂/ Cordicritc, zu bilden.

2. Verfahren zur Herstellung von Ethylenglykol durch Hydrierung von Dimethyloxalat, DMO, unter Verwendung des Katalysators mit monolithischer Struktur, der nach dem Verfahren von Anspruch 1 hergestellt wurde, wobei das Verfahren die folgenden Schritte umfasst:
Einbringen eines Katalysators mit monolithischer Struktur vom Typ Cu/SiC₂/Cordicritc in einen Festbettreaktor, Durchführen einer Reduktionsreaktion in Gegenwart von 20 % H₂/N₂ bei 250 °C für 20 Stunden mit einer Wasserstoffdurchflußrate von 50 ml/min, Einführen von reinem Wasserstoff in den Reaktor nach der Reduktion, Aufrechterhalten einer Reaktionstemperatur bei 250 °C und eines Reaktionsdrucks bei 2,0 MPa, Verdampfen einer Methanollösung, die 20 Gew.-% DMO enthält, in einem Verdampfer, Vorheizen und Einführen von 20 Gew.-% DMO in den Reaktor, wobei die stündliche Flüssigkeitsraumgeschwindigkeit (LHSV) von DMO 1,5 g.mL⁻¹.h⁻¹ und das molare Verhältnis von H₂/Ester 150 beträgt.

## Revendications

1. Procédé de préparation d'un catalyseur à structure monolithique pour la production de l'éthylène glycol par hydrogénation de l'oxalate de diméthyle, comprenant les étapes suivantes :
a) dissoudre 15,5 g de précurseur de cuivre soluble (NO₃)₂-5H₂O avec 150 mL d'eau pour obtenir 0,37 M de solution A;
b) utiliser une solution aqueuse d'ammoniac à 25 % en poids comme précipitant et mélanger la avec la solution A qui est un complexe cuivre-ammoniac ;
c) ajouter un précurseur de silicate, 45 mL de sol de silice à 30 % en poids, dans la solution A, le complexe cuivre-ammoniac, agiter pendant 4 heures, chauffer à 95 °C pour précipiter le cuivre et le silicate, arrêter le chauffage lorsque le pH de la solution est inférieur à 7, filtrer, laver 3 fois avec de l'eau déminéralisée, sécher à 120 °C pendant 12 heures, et calciner un précipité résultant à 450°C pendant 4 heures pour obtenir un catalyseur en poudre B ;
d) presser, granuler et tamiser une partie du catalyseur en poudre B pour former le catalyseur granulaire C à 40 à 60 mailles, mélanger de manière mécanique l'autre partie du catalyseur en poudre B en quantité de 8,0 g, 8,0 g de catalyseur granulaire C, 0,5 g de pseudoboehmite et 50 ml d'eau pour former un catalyseur boueux D, avec une durée de mélange de 2 heures et une vitesse de rotation de 200 tr/min ;
e) imprégner un nid d'abeille en cordiérite de 400 cpsi ayant une granulométrie Φ de 15 × 25 mm, dans le catalyseur boueux D pendant 5 min, souffler le surplus de la boue du véhicule, puis enrober le catalyseur boueux D sur le nid d'abeille en cordiérite par enrobage à trempage, sécher à 120 °C pendant 12 heures et calciner à 450 °C pendant 6 heures pour former un catalyseur monolithique E, soit Cu/SiO₂/cordierite.

2. Procédé de production de l'éthylène glycol par hydrogénation de l'oxalate de diméthyle, DMO, à l'aide du catalyseur à structure monolithique préparé par le procédé selon la revendication 1, le procédé comprenant les étapes suivantes :
mettre un catalyseur à structure monolithique de type Cu/SiO₂/cordierite dans un réacteur à lit fixe, effectuer une réaction de réduction en présence de 20% H₂/N₂ à 250°C pendant 20 heures avec un débit d'hydrogène de 50 mL/min, après la réduction, introduire de l'hydrogène pur dans le réacteur, maintenir une température de réaction à 250°C et une pression de réaction à 2,0 MPa, vaporiser une solution de méthanol contenant 20 % en poids de DMO dans un évaporateur, préchauffer et introduire 20 % en poids de DMO dans le réacteur avec une vitesse spatiale horaire liquide (LHSV) du DMO de 1.5 g.mL⁻¹.h⁻¹et un rapport molaire H₂/ester de 150.
